# EUROPEAN PATENT SPECIFICATION

(11) **EP 2 742 942 B1**
(45) Date of publication and mention of the grant of the patent: **22.05.2019**
(21) Application number: 13193158.6
(22) Date of filing: 15.11.2013
(51) Int. Cl.: A61K 31/455, A61K 8/67, A61Q 9/00

(54) **Niacinamide for inducing generation of antimicrobial peptides**
Niacinamid zum Einleiten der Erzeugung von antimikrobiellen Peptiden
Niacinamide pour induire la production de peptides antimicrobiens

(30) Priority: 14.12.2012 EP 12197301
(43) Date of publication of application: 18.06.2014
(60) Divisional application: 16182135.0
(73) Proprietor: Unilever N.V., 3013 AL Rotterdam (NL); Unilever PLC, Blackfriars London Greater London EC4Y 0DY (GB)
(72) Inventor: Majumdar, Amitabha, 560 066 Whitefield, Bangalore (IN); Mathapathi, Mruthyunjaya Swamy, 560 066 Whitefield, Bangalore (IN); Palanisamy, Bharath, 560 066 Whitefield, Bangalore (IN); Sampatha Kumar, Ramya, 560 066 Whitefield, Bangalore (IN); Tiwari, Jyoti Kumar, 560 066 Whitefield, Bangalore (IN)
(74) Representative: van den Brom, Coenraad Richard

(56) References cited:
- WO-A1-2006/040048
- WO-A1-2011/133692
- WO-A1-2012/137169
- WO-A2-2012/049677
- DE-A1-102009 044 970
- US-A- 4 725 609
- US-A1- 2004 081 672
- DIAMOND, G. ET AL.: "The roles of antimicrobial peptides in innate host defense", CURRENT PHARMACEUTICAL DESIGN, vol. 15, July 2009 (2009-07), pages 2377-2392, XP002694496,

## Description

### Field of the invention

The invention relates to new use of niacinamide for triggering generation of AMPs (anti-microbial peptides) on skin. This has application in improving the immunity of skin, scalp and oral cavity against attack by microorganisms.

### Background of the invention

Skin is the primary line of defense that protects the human body from invading pathogens, like viruses and bacteria. As a primary defense organ, the skin tissue always remains in constant contact with the environment and therefore it has to face and resolve threats and challenges from invading pathogens. The exposed skin surface is not only challenged by pathogenic foreign bacteria, but it also remains in contact and interacts with the resident commensal bacteria. In spite of all these challenges from the foreign and the commensal microbes, healthy skin remains infection free and also the numbers of the resident microflora remains constant. This equilibrium in the interaction between the skin tissue and the microbes is maintained as the skin has sophisticated defense strategy and Anti-microbial peptides (AMPs) form an important part of that.

AMPs form an integral part of the skin's own defense system. AMPs were initially discovered in insects and in animals and ever since their initial discovery AMPs are regarded as promising antimicrobials. AMPs are ubiquitous in nature and they typically exhibit a broad spectrum of activity against invading bacteria, fungi, enveloped viruses and parasites (Braff and Gallo, 2006. AMPs are generally short peptides and in humans about 90 different AMPs are reported to be present. AMPs in general have two major physical features and they are - a) cationic charge and b) a significant proportion of hydrophobic residues. The cationic charge of the AMPs promotes selectivity for negatively charged microbial cytoplasmic membranes whereas the hydrophobicity facilitates interactions with the cell membrane of the microbial species.

The present inventors have been working to provide hygiene benefits to consumers through the route of enhancing the AMP levels in the skin. They wish to provide this through use of natural molecules which are perceived by the consumers to be more skin friendly and thereby less harsh. To achieve this goal various actives were tested and after extensive research arrived at finding the use of Niacinamide or Vitamin B3 for enhancing AMP levels on the skin.

Niacinamide or Vitamin B3 is a well known skin lightening active used in several products for skin care. Niacinamide is also reported to have various other properties like prevention of cellulite, treating skin irritation and for improving absorption of water on to skin (reported in patent publications EP1063963, EP1063994, EP1063965 to name a few). Niacinamide is also used in the treatment of many inflammatory skin conditions like Acne vulgaris, Psoriasis and Atopic dermatitis (-Niren,N.M. (2006) Pharmacologic doses of nicotinamide in the treatment of inflammatory skin conditions: a review. Cutis 77: 11-16.).

WO11133692 (Cedars-Sinai Medical Centre) discloses the important role of C/EBPe in innate immune response against pathogens. Specifically, it has been shown that in the absence of functional C/EBPe, mice are severely impaired in their ability to clear *S*. *aureus* infection. Neutrophils are particularly affected, and susceptibility to *S. aureus* can be rectified by treatment with interferon-gamma (IFN-γ). Importantly, increased activity of C/EBPe, either by induced overexpression of C/EBPE or by application of nicotinamide or an analog, derivative or salt thereof, dramatically enhances immune killing of *S*. *aureus* and leads to amelioration of infection.

WO12137169 (L'Oréal) discloses the use of cosmetic compositions comprising a full bacterial lysate for treating dandruff on the scalp. The induction of antimicrobial peptides in the scalp is triggered by this treatment: however, WO12137169 does not disclose nor suggest the use of niacinamide for this purpose.

The present inventors have found that on application of niacinamide on skin (which could be skin per se or scalp or oral cavity), the active induces generation of AMPs which is known to be an important step in improving the immunity of the skin against attack by microorganisms. Thus the benefit obtained by a consumer is that the skin is protected against germs which may attack in the future by application of a composition comprising niacinamide.

### Summary of the Invention

The invention provides for use of niacinamide for inducing secretion of anti-microbial peptides (AMPs) when applied on external surface of human body.
Accordingly, it also provides niacinamide for prophylactically inducing secretion of anti-microbial peptides (AMPs) when applied on an external surface of the human body.

### Detailed Description of the Invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

"Skin" as used herein, is meant to include the external surface of mammals, especially humans and includes skin, scalp, hair and oral cavity. The use could be by way of incorporating niacinamide in a leave-on or in a rinse off product, and includes any product applied to a human body primarily for lightening skin but may also improve appearance, cleansing, odor control or general aesthetics. The use is preferably by way of incorporation in a leave-on composition. The composition can be in the form of a liquid, lotion, cream, foam, scrub, gel, soap bar or toner, or applied with an implement or via a face mask, pad or patch. Non-limiting examples of such compositions include leave-on skin lotions and creams, shampoos, conditioners, shower gels, toilet bars, antiperspirants, deodorants, depilatories, lipsticks, foundations, mascara, sunless tanners and sunscreen lotions.

Niacinamide has the structure as given below

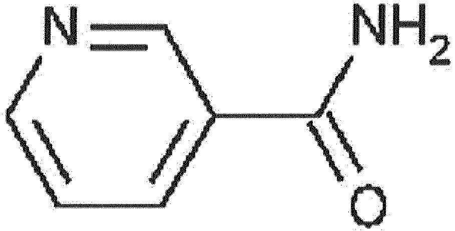

The niacinamide for use in the present invention induces secretion of AMPs from keratinocytes. The AMPs thus secreted provides for improving the immunity of the external surface of the body. The external surface includes skin, scalp or oral cavity.

It has been found by way of the present invention that niacinamide activates keratinocytes, which are the major cells in the skin epidermis to provide the benefits of the present invention viz. inducing secretion of anti-microbial peptides (AMPs). This causes niacinamide to boost protection shield against germs. Niacinamide therefore provides protection for the body against infections by boosting the body's own defence. In other words, the active primes the body surface for germ protection. The advantage of this is that it provides long-lasting protection e.g. up to 24 hours of protection against germs.

The composition in which niacinamide is used as per the present invention may comprise the following preferred features. Niacinamide is preferably present in 0.1 to 5%, more preferably 0.5 to 5%, further more preferably 0.5 to 3%, and optimally 1.0 to 3.0% by weight of the composition. The composition preferably comprises a cosmetically acceptable base. The cosmetically acceptable base is preferably a cream, lotion, gel or emulsion.

Personal care compositions may be prepared using different cosmetically acceptable emulsifying or non-emulsifying systems and vehicles. Preferred cosmetically acceptable bases comprise 1 to 25% fatty acid. A further preferred aspect provides for inclusion of 0.1 to 10% soap. A highly suitable base is a cream. Vanishing creams are especially preferred. Vanishing cream bases generally comprise 5 to 25% w/w fatty acid and 0.1 to 10% w/w soap. Vanishing cream base gives a highly appreciated matty feel to the skin. C₁₂ to C₂₀ fatty acids are especially preferred in vanishing cream bases, further more preferred being C₁₄ to C₁₈ fatty acids. The most preferred fatty acid is stearic acid. The fatty acid may also be a mixture of palmitic and stearic acid. The fatty acid in the composition is more preferably present in an amount in the range of 5 to 20% w/w of the composition. Soaps in the vanishing cream base include alkali metal salt of fatty acids, like sodium or potassium salts, most preferred being potassium stearate. The soap in the vanishing cream base is generally present in an amount in the range of 0.1 to 10%, more preferably 0.1 to 3% w/w of the composition. The personal care composition when formulated as a vanishing cream preferably comprises 60 to 85%, more preferably 65 to 80% w/w water. Water is generally present in many compositions prepared as per the invention and is generally present in 40 to 85% by weight of the composition.

The composition of the invention may comprise other optional ingredients like skin lightening agents, and one or more UV sunscreens. The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition may optionally be delivered as a deodorant. By a deodorant is meant a product in the stick, roll-on, or propellant medium which is used for personal deodorant benefit e.g. application in the under-arm area which may or may not contain anti-perspirant actives.

Deodorant compositions can generally be in the form of firm solids, soft solids, gels, creams, and liquids and are dispensed using applicators appropriate to the physical characteristics of the composition.

The compositions may comprise a wide range of other optional components. The CTFA Cosmetic Ingredient Handbook, Second Edition, 1992, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

The composition is formulated in any known format, more preferred formats being creams or lotions.

The use of niacinamide by way of the present invention is non-therapeutic.

The invention is now further described by way of the following non-limiting examples.

### Examples

### Examples 1 to 6: Examples to prove that niacinamide induces LL-37 gene expression but does not change the psoriasin expression

The following samples as shown in Table - 1 were prepared for measuring their effect on psoriasin expression and the psoriasin AMP (LL-37) expressed.

**Table - 1**

| Example | Sample |
|---|---|
| 1 | Control for LL-37 gene expression |
| 2 | Positive control LPS (25 ↘g/ml) for LL-37 gene expression |
| 3 | Niacinamide (1.22 mg/ml) for LL-37 gene expression |
| 4 | Control for psoriasin gene expression |
| 5 | Positive control LPS (25 ↘g/ml) for psoriasin gene expression |
| 6 | Niacinamide (1.22 mg/ml) for psoriasin gene expression |

The material used for and the procedure used for measuring the respective gene expression is given below.

### Materials

Normal human neonatal epidermal primary keratinocytes (NHEK), Keratinocyte growth media (KGM) and growth supplements, Antibiotics (Penicillin and Streptomycin), *E*. *coli* strain (ATCC strain 10536), Lipopolysaccharide (LPS) from Escherichia coli 055:B5 strain, 1X Phosphate buffer saline, 10mM sodium phosphate buffer, Niacinamide, Sterile cups (both ends are open-barrel shape, open end area is 2.27 cm² and rods are round tip made of hard Teflon (Appendix-1), Vaseline ingredients (Appendix-2), Psoriasin (S100A7) (CircuLex) and LL-37 ELISA kit (Hycult), Macconkey agar medium, TSA medium (Unilever research report No: BL 11 0086).

### Stimulation of human primary skin keratinocvtes

Human primary keratinocytes prepared from neonatal foreskin were obtained from (Lonza India) and cultured in serum-free keratinocyte growth medium with defined growth supplements (Invitrogen). Keratinocytes were cultured in 12 and 24 well tissue culture plates (BD Falcon) and treated cells at a confluence of 60-70%. Keratinocytes stimulation was performed in KGM with growth supplement and all experiment was done between passages 3 to 5.

### RNA isolation and quantitative real-time polymerase chain reaction (PCR)

NHEKs were cultured in 12-well plates (BD, Falcon) at 6 × 10⁴ cells per well. After 24h of incubation, 50% media was replaced with fresh media. On day 3, cells were treated with different concentrations of Niacinamide with fresh media for 16-18 hours. Cells were harvested in RLT buffer (Qiagen) and samples were stored at -70°C till used for RNA isolation.

Total RNA was isolated using RNeasy Mini kit from Qiagen, according to the manufacturer's instructions. The purity and RNA concentration was checked using NanoDrop (Thermo scientific) instrument. The RNA samples were loaded on 1% agarose gel and the quality of extraction was observed using UV-trans-illuminator (Bangalore Genie, India). RNA samples were aliquoted and stored at -70°C. An aliquot of total RNA (250 to 500ng) was used to make cDNA by using iScript cDNA synthesis kit (Bio-Rad, USA). The cDNA synthesis program was 65°C for 5min followed by 25°C for 5 minutes. The cDNA synthesis was done at 42°C for 30 minutes, final denatured at 85°C 5min followed by 4°C until removed.

Real time PCR was carried out using SYBR green method as per the manufacturer's (Bio-Rad, USA) instructions using thermal controlled Chromo4 machine (Bio-Rad, USA). 5µl of 1:10 diluted sample of cDNA and 1.5pmole of specific primers were used in each reaction by using a standardized PCR program (i.e. 94°C for 5 minutes followed by 50 cycles of "94°C for 15 seconds, 60°C for 30sec, 72°C for 30 seconds) and results were analysed by using **2^{-ΔΔCT}** method.

### Psoriasin and LL-37 ELISA

Primary keratinocytes were seeded in 12-well plates (BD falcon). At 60 to 70% confluency, cells were treated with different concentration of Niacinamide for 72h. After 72 hours of incubation cell culture supernatant was stored at -70°C until used for ELISA. All ELISA reagents and samples were brought to room temperature prior to the assay. Cell culture supernatant samples were centrifuged at 5000 rpm for 10 minutes before use. 100 ↘l of undiluted sample of cell culture supernatant was used for checking the psoriasin level by using ELISA kit from CircuLex. The clinical samples were diluted in to 1:25 in dilution buffer and used 100↘l from diluted samples for checking psoriasin level by ELISA. In case of LL-37, 100 ↘l of undiluted samples from *in vitro* as well as clinical samples were used for checking LL-37 level by ELISA (Hycult Biotech).

The gene expression of Psoriasin and LL-37 AMP in keratinocytes treated with Niacinamide for 18 hours. LPS (20µg/ml) used as a positive control, to trigger the gene expression of Psoriasin and LL-37 AMPs in primary keratinocytes after 18 hours was studies using the procedure give above. The results in terms of fold change is given in the Table -2 below.

**Table -2**

| Example | Sample | Fold change |
|---|---|---|
| 1 | Control for LL-37 gene expression | 1.0 |
| 2 | Positive control LPS (25 ↘g/ml) for LL-37 gene expression | 3.9 |
| 3 | Niacinamide (1.22 mg/ml) for LL-37 gene expression | 2.4 |
| 4 | Control for psoriasin gene expression | 1.0 |
| 5 | Positive control LPS (25 ↘g/ml) for psoriasin gene expression | 2.3 |
| 6 | Niacinamide (1.22 mg/ml) for psoriasin gene expression | 1.1 |

The data in table 2 above indicates that Niacinamide was unable to induce psoriasin gene expression even at a very high concentration (1.22 mg/ml), but surprisingly, niacinamide was able to induce the secretion of psoriasin AMP which was detected using ELISA technique.

### Examples 7 to 12: In vivo experiments with skin lotions containing niacinamide to prove induced secretion of psoriasin and LL-37 on skin

To confirm the findings from the *in vitro* experiments, the effect of Niacinamide on healthy human volunteers was tested *in vivo.* The volunteers were asked to apply a lotion with and without niacinamide (3%) on their forearm twice a day. After 7 days of application, the spots were extracted in PBS and ELISA was performed for detection of Psoriasin and LL-37 AMPs.

### In Vivo study procedure

### E. coli culture preparation

Glycerol stock of *E*. *coli* (10536) was inoculated in 30 ml TSB medium and culture was incubated overnight at 37°C in shaker incubator. Overnight culture was sub cultured on TSA slants and incubated overnight at 37°C then the slants were stored at 4°C (once in 15 days, slants were prepared freshly).

*E. coli* culture from slant was sub cultured on TSA plate and incubated overnight at 37°C. Overnight grown plate culture of *E*. *coli* was suspended in 6 to 7 ml of Sodium phosphate buffer (10mM). Density of culture was adjusted to 1 OD₆₂₀ using spectrophotometer and used for clinical studies.

### Cup Scrub method of extraction

This technique is included in the ASTM method, E2752 - 10 (Standard guide for evaluation of residual effectiveness of antibacterial). The cup and rod were made up of Teflon and the area of the cup is 2.84 cm². The cup was used to hold the buffer on skin and rod was used for scrubbing, for better recovery of skin AMP and/or residual bacteria from skin. The duration of scrubbing was 1min. The extracted sample was collected in 1.5ml eppendorf tube for analysis (Appendix-1).

### Estimation of psoriasin and LL-37 AMPs and enumeration of residual E. coli from human skin by cup scrub method

10 µl of 1 OD₆₂₀ culture of *E*. *coli* was applied on 2.27 cm² area on the marked area on the forearm (No cream, Base cream and Niacinamide cream applied spot). The culture was spread uniformly on each spot. After 15 min contact time, the samples were extracted by cup scrub method in 1 ml PBS. The samples were collected in a sterile microfuge tube.

In the extracted sample, 800↘l was used to enumerate the residual E. *coli* by standard microbiological method (plated 100↘l of undiluted, -1 and -2 diluted samples on MacConkey agar plate by spread plate method). The remaining 200↘l of the sample was used to analyse the psoriasin and LL-37 level by ELISA using standardized kits. The neat sample was used to check the level of LL-37 in the samples. In case of psoriasin the samples were diluted in to 1:25 in dilution buffer (which was provided along with the kit) and used 100↘l of diluted samples for analysis.

### In vivo study design

1. Volunteers were asked come to the study site after bath every day morning.
2. An area of 6.25 cm² area (2.5 x 2.5cm) was marked on the inner forearm using a marker pen and scale. Three areas were marked were marked on the forearm, excluding the wrist (5 cm from the base of the palm). (One marker pen was given to each volunteer to mark the area if it becomes dull or gets erased).
3. Aliquots of 62.5mg of the formulations were weighed in sterile containers (35mm plastic round dishes) and were given to the volunteers.
4. Volunteers were asked to apply the formulations (62.5 mg/6.25 cm² area) in the delineate spots on the forearm. (The order of application was base formulation followed by formulations containing actives). The formulations were spread over the area for -30 s each.
5. After application of each formulation the finger was wiped with sterile tissue paper.
6. Similar aliquots of 62.5 mg formulation were weighed in sterile 35 mm plastic petri dishes and were sealed with parafilm and given to each volunteer to apply in the night (before sleep).
7. Steps 3 to 6 were repeated for 7 days for a 7 day study. In case of one day study the assessment was done on the second day.
8. On day 8 volunteers were asked to come to the study site in the morning without washing their forearms.
9. The forearms of the volunteers were washed (by the investigators) with non-antibacterial soap (Lux soap).
10. Excess water was removed by patting dry using sterile tissue paper.
11. The volunteers were asked to wait for 6 h without touching the forearm.
12. A circular area of 2.27 cm² was marked on each cream application spot using a template and 10µl of *E*. *coli* 10536 culture corresponding to 1X10⁸ to 1X10⁹cells/ml was added to each circular area and spread uniformly.
13. After 15 min of contact time, *E*. *coli was* recovered by cup-scrub method using 1ml of 1X PBS. Duration of extraction was 1min.
14. Samples were analysed for bacterial count by spread plate method on MacConkey's agar.

The residual log of E,Coli in the forearm area in the various experiments are summarized in the table - 3 and Table - 4 below:

**Table -3: Residual log of E.Coli measured after one day of applying a composition (n= 28)**

| Example | Sample | Fold change |
|---|---|---|
| 7 | Basal residual log of E. Coli | 4.21 |
| 8 | Residual log when lotion without niacinamide was applied | 3.44 |
| 9 | Residual log when lotion with 3% niacinamide was applied | 2.31 |

**Table - 4: Residual log of E.Coli measured after 7 days of applying a composition (n= 34)**

| Example | Sample | Fold change |
|---|---|---|
| 10 | Basal residual log of E. Coli | 4.41 |
| 11 | Residual log when lotion without niacinamide was applied | 3.56 |
| 12 | Residual log when lotion with 3% niacinamide was applied | 2.39 |

The data in Table - 3 above indicates that a composition comprising niacinamide is capable of reducing bacteria even after one day of use while Table -4 indicates that this effect is retained for a long period of time (seven day application).

### Example -13: Experiment to determine the types and amounts of anti-microbial peptides (AMPs) secreted due to induction by niacinamide.

The following procedure was used to determine the AMPs induced as a consequence of niacinamide on keratinocte cells.
1. Primary keratinocytes were seeded in 12 well plates with 1ml of KGM complete media. (Seeded 60,000 cells/well at Passage-4).
2. Plates were incubated in the CO₂ incubator for 48 hours.
3. After 48 hours of incubation, cells were treated with and without Niacinamide 1 mg/ml concentration in duplicate wells (fresh media was used for treatment).
4. After 72 hours of incubation, cell culture supernatant was transferred in to 1.5 ml eppendorf tubes respectively.
5. Samples were then passed through 20 kDa cutoff filters to remove high molecular weight proteins.
6. Eluted samples, containing <20kDa proteins were transferred to 15ml sterile centrifuge tubes.
7. Then, chilled acetone (HPLC grade) was added to the eluted samples (1 ml of sample:4ml of acetone)
8. Tubes were transferred to -20°C for 24 hours to precipitate the proteins.
9. After 24 hours, tubes were centrifuged at 14000g for 40 minutes at 4°C.
10. The supernatant was discarded and the protein pellet was analysed using mass spectrometry for proteins.

The data on the increased levels of secretion of various AMPs is tabulated in Table - 5. The increased level of secretion of AMPs is measured by a PSM value (a peptide spectrum match scoring function) which is a relative measure of abundance of the peptide in the secretome.

**Table - 5**

| AMP secreted | PSM value without niacinamide treatment (Control sample) | PSM value with niacinamide treatment |
|---|---|---|
| Lactoferrin | 1 | 7 |
| Lipocalin -2 | 1 | 4 |
| S-100-A8 | 1 | 3 |
| S-100-A11 | 2 | 5 |
| Elafin | 1 | 3 |
| Lysozyme C | 2 | 4 |
| S100-A9 | 2 | 4 |
| Lipocalin-1 | 0 | 2 |

The data in Table -5 indicates that there is significantly high level of secretion of various AMPs as a consequence of use of niacinamide.

### Examples 14-17: Human volunteer study on ability of the skin to defend itself against infections

An experiment using the following protocol was conducted:
1. Volunteers were given Lux™ soap for use and asked not to use any other soap or moisturizers on their forearm for 7 days before the start of the study.
2. Volunteer were be asked to apply Vaseline™ healthy white base cream and Vaseline™ healthy white formulated with Niacinamide (1%, 2% and 3%) at various spots on the forearm.
3. Volunteers were asked to repeat step 1 for two times per day for 1 day, morning after bath and night just before sleep. The volunteers were asked not to wash the forearms after application of formulations at night.
4. On day 2, the forearms were washed by the investigator with a non-antibacterial soap bar and asked the volunteers to wait for 6 hours under controlled conditions (24 ±2°C and 45%±5% RH)
5. After 6 hours of waiting, ∼10⁶ *E*. *coli* were applied on the forearm to each spot separately. The contact time of *E*. *coli* on skin was 15 minutes.
6. After 15 minutes, the sample was extracted from the forearm using the cup-rod method.
7. *E*. *coli was* counted using standard microbiological method.

The data on the *E.Coli* count for the various treatments is summarized in Table - 6.

**Table -6**

| Example | Sample | Residual log of *E. Coli* |
|---|---|---|
| 15 | Control | 4.3 |
| 16 | 1% niacinamide | 3.5 |
| 17 | 2% niacinamide | 3.0 |
| 18 | 3% niacinamide | 2.7 |

The data in Table-6 indicates that use of niacinamide boosts skin's inherent immunity against invading bacteria.

The invention thus provides for a new use of niacinamide not known hitherto which is to induce secretion of anti-microbial peptides (AMPs) when applied on external surface of human body.

## Claims

1. Non-therapeutic use of niacinamide for inducing secretion of anti-microbial peptides (AMPs) from keratinocytes when applied on an external surface of the human body.

2. Non-therapeutic use as claimed in claim 1 for improving the immunity of said external surface.

3. Non-therapeutic use as claimed in claim 1 or 2 wherein said external surface includes skin, scalp or oral cavity.

## Patentansprüche

1. Nichttherapeutische Verwendung von Niacinamid zum Einleiten der Sekretion antimikrobieller Peptide (AMPs) durch Keratinozyten bei Applikation auf eine externe Oberfläche des menschlichen Körpers.

2. Nichttherapeutische Verwendung nach Anspruch 1 zur Verbesserung der Immunität der externen Oberfläche.

3. Nichttherapeutische Verwendung nach Anspruch 1 oder 2, wobei die externe Oberfläche Haut, Kopfhaut oder Mundhöhle umfasst.

## Revendications

1. Utilisation non thérapeutique du niacinamide pour induire la sécrétion de peptides antimicrobiens (PAM) depuis des kératinocytes quand il est appliqué sur une surface externe du corps humain.

2. Utilisation non thérapeutique selon la revendication 1 pour améliorer l'immunité de ladite surface externe.

3. Utilisation non thérapeutique selon la revendication 1 ou 2 où ladite surface externe inclut la peau, le cuir chevelu ou la cavité orale.
